# EUROPEAN PATENT APPLICATION

(11) **EP 0 633 540 A2**
(43) Date of publication of application: **11.01.1995**
(21) Application number: 94304452.9
(22) Date of filing: 20.06.1994
(51) Int. Cl.: G06F 19/00

(54) **Control system for the alarms of a patient monitoring apparatus**

(30) Priority: 06.07.1993 FI 933098
(71) Applicant: INSTRUMENTARIUM CORPORATION, SF-00510 Helsinki (FI)
(72) Inventor: Rantala, Börje, SF-00200 Helsinki (FI)
(74) Representative: Charlton, Peter John

(57) **Abstract**

The invention relates to a control system for the alarms of a patient monitoring apparatus, comprising a user interface (2, 7) for feeding the proceedings of an operation and physiological data of a patient into the electronic memory of a monitoring apparatus (1). The patient monitoring apparatus (1) is fitted with a surgical report interface which provides information about the varying stages of an operation, such information being used for controlling the alarm functions of the monitoring apparatus (1) itself. The surgical report software can be built in integral linkage with the patient monitoring apparatus (1).

## Description

The present invention relates to a control system for the alarms of a patient monitoring apparatus, comprising a user interface for feeding the proceedings of an operation and physiological data of a patient into the electronic memory of a monitoring apparatus.

An object of the invention is to further develop such a system in a manner that the control for the alarms of a patient monitoring system can be performed on the basis of data included in a patient information system.

In the operating theatre and intensive care environment, one of the major limitations in the reliability and accuracy of alarms issued by a patient monitoring system is the fact that the monitoring device must base an alarm decision on electronic signals containing just limited information. Neither is it often possible to make sure that the connection to a patient is in order; for example, the sampling tube for respiratory gases may sag on the floor and, thus, no gas content information will be obtained.

Both the parameters to be monitored and the importance thereof as alarm sources vary widely according to which stage of an operation is in progress. At the initial stage, for example, the gas monitoring is difficult and often unnecessary as the patient has not been intubated. Immediately after the intubation, however, the gas monitoring is of extreme importance. Likewise, when the circulation of a patient is supported by a cardiac respiratory machine, the alarm criteria are essentially different; after all, at that time the heart is deliberately stopped, a situation that would otherwise lead to a top priority alarm.

It is known as such that a patient monitoring device can be informed by the user as to which stage of an operation is in progress or which type of alarm logic would be desirable. However, this type of system is inconvenient as it means extra work for the user and is easily overlooked.

The automatic identification of an operative stage is not generally successful, either.

The events of operative proceedings are routinely documented in a surgical report. The above-described problem is overcome by the invention in such a manner that a patient monitoring device is fitted with a surgical report interface which provides information about the varying stages of an operation, such information being used for controlling the alarm functions of the monitoring device itself. In other words, the automatically or routinely performed preparation of a surgical report is linked with a patient monitoring apparatus and these routine documentations of operative proceedings are harnessed for the control of an alarm logic.

The surgical report includes as an integral part thereof the documentation of surgical proceedings, especially significant proceedings, which is also in principle intended to be effected in "real time", i.e. within about 5 minutes from the procedure.

The invention will be further illustrated with reference to the accompanying drawings, wherein:

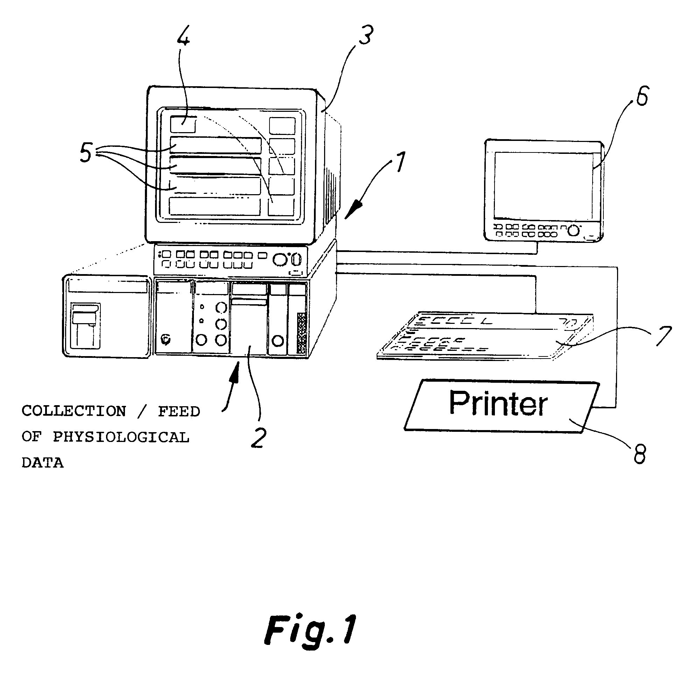

- Fig. 1: depicts a general view of a patient monitoring apparatus of the invention; and

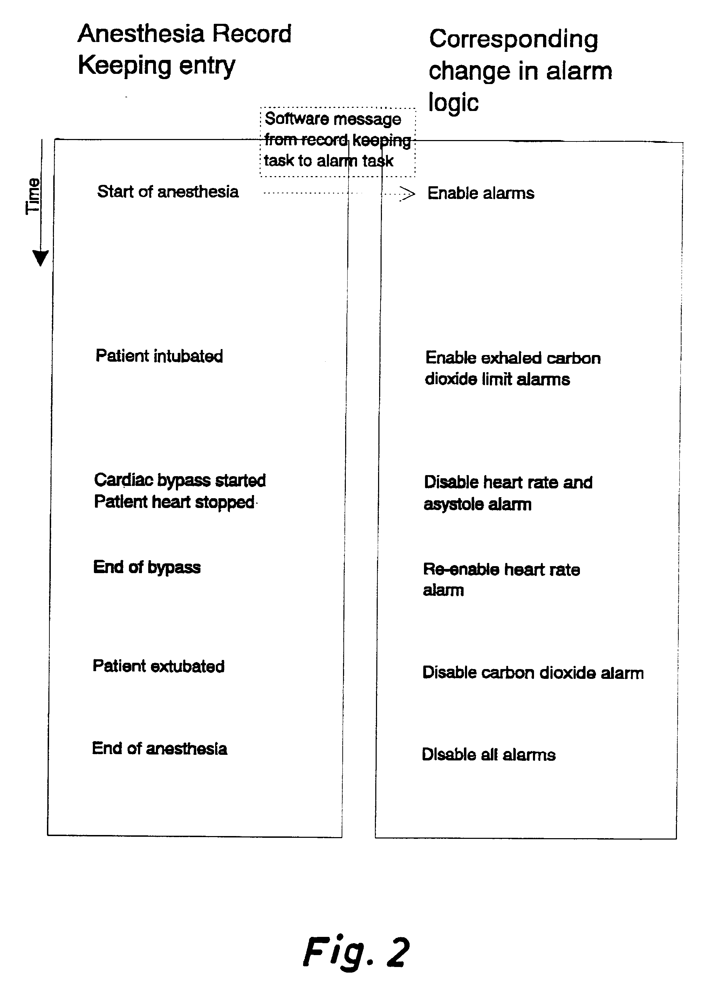

- Fig. 2: shows an example of software messages from record keeping task to alarm task.

A monitoring apparatus 1 includes a data processing unit 2 provided with interfaces for electrodes or sensors to be connected to a patient in view of collecting physiological information about a patient and feeding such information into the electronic memory of the data processing unit 2 of the monitoring apparatus or device. The monitoring device is provided with a display 3 which may include e.g. an alarm display 4 and display sections 5 for the physiological data of a patient.

In addition, the data processing unit 2 can be fitted with an optional display 6 for anesthetic information. A keyboard 7 can be used for feeding patient information (name, social security ...) as well as separately recorded developments in the progress of anesthesia into the electronic memory of the unit 2. This information is transferred to an anesthesia record. The monitoring results obtained on the basis of the physiological data of a patient transfer electrically onto an automatic anesthesia record, which is shown on the display 3 or 6 during anesthesia. Generally, the anesthesia record can be printed at any time from the electronic memory of unti 2 onto a printer 8 or a recorder. In addition, an electronic copy of the record is generally stored e.g. on a computer diskette.

Thus, The routine documentation of surgical proceedings includes the recording of essential operative stages, e.g. the assignment of circulation to a cardiac respiratory machine, said recording being stored in the electronic memory of the patient monitoring apparatus 1. Hence, this information can be used by the monitoring device 1 for the control of its own alarm logic. When the monitoring device has been informed this way e.g. of the fact that the heart of a patient has been deliberately stopped, the possibility of triggering an alarm on the basis of a cardiac arrest is automatically eliminated.

When the routine documentation events of surgical proceedings are harnessed for controlling the alarm logic of the apparatus, the result will be a better informed alarm logic with no extra inconvenience in terms of using the apparatus. In practice, the surgical report software is preferably built in integral linkage with the patient monitoring apparatus 1.

## Claims

1. A control system for the alarms of a patient monitoring apparatus, comprising a user interface (2, 7) for feeding the proceedings of an operation and physiological data of a patient into the electronic memory of a monitoring apparatus (1), **characterized** in that the patient monitoring apparatus (1) is fitted with a surgical report interface which provides information about the varying stages of an operation, such information being used for controlling the alarm functions of the monitoring apparatus (1) itself.

2. A system as set forth in claim 1, **characterized** in that the surgical report software is preferably built in integral linkage with the patient monitoring apparatus (1).
